(19) Europäisches Patentamt
European Patent Office
Office européen des brevets

(11) Publication number: **0 331 821 B1**

(12) **EUROPEAN PATENT SPECIFICATION**

(45) Date of publication of patent specification: **19.05.93**

(51) Int. Cl.5: **C07K 15/14**, C12P 21/00, C08B 37/00, A61K 37/02

(21) Application number: **88301602.4**

(22) Date of filing: **25.02.88**

The file contains technical information submitted after the application was filed and not included in this specification

(54) **Antiviral agent.**

(30) Priority: **03.02.88 JP 22998/88**

(43) Date of publication of application:
**13.09.89 Bulletin 89/37**

(45) Publication of the grant of the patent:
**19.05.93 Bulletin 93/20**

(84) Designated Contracting States:
**BE DE FR GB IT NL**

(56) References cited:
**EP-A- 0 058 093**
**FR-A- 2 405 298**
**US-A- 4 051 314**

**CHEMICAL ABSTRACTS, vol. 96, no. 11, 15th March 1982, page 467, abstract no. 84105f, Columbus, Ohio, US; & JP-A-81 112 902 (WAKAMOTO PHARMACEUTICAL CO., LTD) 05-09-1981**

(73) Proprietor: **KUREHA KAGAKU KOGYO KABUSHIKI KAISHA**
**1-9-11, Nihonbashi, Horidome-cho**
**Chuo-ku Tokyo 103(JP)**

(72) Inventor: **Muto, Shigeaki**
**3-23-2 Higashi-Horikiri Katsushika-ku**
**Tokyo(JP)**
Inventor: **Ohhara, Minoru**
**19-25 Fujimi-cho Itabashi-ku**
**Tokyo(JP)**
Inventor: **Kakuchi, Junji**
**6-16-30-406 Minami-Karasuyama**
**Setagaya-ku**
**Tokyo(JP)**
Inventor: **Yoshikumi, Chikao**
**2-19-46 Higashi Kunitachi-shi**
**Tokyo(JP)**
Inventor: **Takahashi, Masaaki**
**1-5-33-314 Takanawa Minato-ku**
**Tokyo(JP)**

(74) Representative: **Woods, Geoffrey Corlett et al**
**J.A. KEMP & CO. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

CHEMICAL ABSTRACTS, vol. 108, no. 15, 11th April 1988, page 345, abstract no. 127418w, Columbus, Ohio, US; K. HIROSE et al.: "A biological response modifier, PSK, inhibits revese transcriptase in vitro", & BIOCHEM. BIOPHYS. RES. COMMUN., 1987, 149(2), 562−7

Tsukagoshi et al. GANN 65, 557 (1974);

Tochikura et al. Biochem. Biophys. Res. Comm. 2, 726 (1987)

## Description

The present invention relates to a specific protein-bound polysaccharide derived from fungi belonging to Coriolus of Basidiomycetes, to the preparation of the protein-bound polysaccharide and to pharmaceutical compositions containing it.

Azido-3'-deoxythymidine (AZT) has already been used clinically as an anti-AIDS drug. This drug has a side effect of inhibiting the mitosis of normal cells. Though vaccines have been heretofore used as antiviral agents, they are not effective as an anti-AIDS drug.

As protein-bound polysaccharides derived from fungi of Basidiomycetes and processes for producing such polysaccharides, the following have been proposed.

JP-A-55-23,271 (1980) discloses a protein-bound polysaccharide possessing a molecular weight in the range of 5,000 to 300,000 as measured by the ultracentrifugal method; exhibiting positive color reactions characteristic of saccharides in the $\alpha$-naphthol sulfuric acid reaction, the indole sulfuric acid reaction, the anthrone sulfuric acid reaction, the phenol sulfuric acid reaction and the tryptophane sulfuric acid reaction; exhibiting positive color reactions characteristic of the peptide bond and amino acids in the Lowry-Folin method and ninhydrin reaction after hydrochloric acid hydrolysis; possessing absorption regions of 0.9 ± 0.1 ppm, 1.2 ± 0.1 ppm, 2.0 ± 0.1 ppm, 4.5 ± 0.1 ppm and 4.7 ± 0.1 ppm and a broad absorption region of 3.0 to 4.4 ppm as measured by proton nuclear magnetic resonance spectroscopy; having a ratio of saccharide moiety to protein moiety in the range of 55/45 to 95/5 provided that the proton strength of the protein moiety falls in the range of 0.5 to 2.5 ppm and the proton strength of saccharide moiety in the range of 2.5 to 6.0 ppm; having the saccharide moiety composed of $\beta$-glucan; showing no absorption attributed to an $\alpha$-glucan of 4.9 to 6.0 ppm; and having the protein moiety composed of aspartic acid, threonine, serine, glutamic acid, proline, glycine, alanine, cysteine, valine, methionine, isoleucine, leucine, tyrosine, tryptophane, phenylalanine, lysine, histidine and arginine. This document also discloses a process for producing the aforementioned protein-bound polysaccharide comprising preculturing a seed culture of a Basidiomycetous fungus thereby inducing growth of fungal lichen on the surface of a culture medium, homogenizing the fungal lichen with physiological saline solution thereby preparing a seed culture for production culture, cultivating the seed culture by stationary or submerged culture thereby obtaining a mycelium, extracting the mycelium with hot water or an aqueous solvent such as dilute alkaline solution, concentrating the extract after removal of residue therefrom, salting out the concentrate with ammonium sulfate or subjecting to ultrafiltration thereby removing low molecular substances therefrom and obtaining a refined concentrate, adding an amount of ammonium sulfate equivalent to a saturation degree of 25% to the resultant concentrate thereby inducing precipitation, removing the precipitate from the resultant solution, adding an amount of ammonium sulfate equivalent to a saturation degree of 40% to the resultant solution, collecting the precipitate consequently formed in the solution, desalting a solution of the precipitate by dialysis, subjecting the resultant desalted solution to DEAE cellulose column, eluting the adsorbate with an aqueous 1 mol sodium chloride solution, adding an amount of ammonium sulfate equivalent to a saturation degree of 40% to the eluate, collecting the precipitate consequently formed in the resultant reaction solution, and desalting a solution of the precipitate by dialysis.

US-A-4 271 151 discloses an antitumor agent suitable for oral administration to a mammal having cancer, said antitumor agent being capable of retarding the growth of said cancer. The agent comprises an effective amount of a protein-bound polysaccharide obtained by extraction of a mycelium or fruit body of a Coriolus of Polyporaceae of Basidiomycetes. The protein-bound polysaccharide has a molecular weight within the range of 5,000 to 300,000 as measured with ultracentrifugation; gives positive color reactions characteristic of saccharides with $\alpha$-naphthol-sulfuric acid, indolesulfuric acid, anthrone-sulfuric acid, phenol-sulfuric acid, and tryptophane-sulfuric acid; gives a positive ninhydrin color reaction characteristic of amino acids after hydrochloric acid hydrolysis and a positive color reaction in the Lowry-Folin test characteristic of peptide linkage; said protein-bound polysaccharide having absorption regions, as measured by proton nuclear magnetic resonance spectra, at 0.9 ± 0.1 ppm, 1.2 ± 0.1 ppm, 2.0 ± 0.1 ppm, 4.5 ± 0.1 ppm and 4.7 ± 0.1 ppm, with broad absorption at 3.0-4.4 ppm; said protein-bound polysaccharide having a polysaccharide portion/protein portion ratio within the range of 55/45 to 95/5 provided that the proton strength of the protein portion is at 0.5-2.5 ppm and that of the polysaccharide portion is at 2.5-6.0 ppm; said polysaccharide portion being composed of $\beta$-glucan and showing no absorption attributed to $\alpha$-glucan at 4.9-6.0 ppm; said protein portion being composed of aspartic acid, threonine, serine, glutamic acid, proline, glycine, alanine, cysteine, valine, methionine, isoleucine, leucine, tyrosine, tryptophane, phenylalanine, lysine, histidine and arginine.

JP-A-56-46,481 (1981) discloses a polysaccharide possessing a molecular weight in the range of 5,000 to 300,000 as measured by the ultracentrifugal method; exhibiting positive color reactions characteristic of saccharides in the α – naphthol sulfuric acid reaction, the indole sulfuric acid reaction, the anthrone sulfuric acid reaction, the phenol sulfuric acid reaction, and the tryptophane sulfuric acid reaction; having an elementary analysis of 43.5 to 45.3% of carbon, 5.7 to 6.7% of hydrogen, and the balance of oxygen; exhibiting a specific rotation, $[\alpha]_D^{25}$ , in the range of 70° to 180°; showing a characteristic absorption at 840 $cm^{-1}$ in the infrared absorption spectrum; exhibiting absorption regions of 3.7 ± 0.1 ppm, 3.8 ± 0.1 ppm, 5.0 ± 0.1 ppm, and 5.4 ± 0.1 ppm; exhibiting solubility in water and insolubility in chloroform and hexane; and possessing D – glucose as a main component saccharide.

US – A – 4 614 733 discloses a polysaccharide having a molecular weight of from 5,000 to 300,000 as determined by ultracentrifugation; giving color reactions characteristic of saccharides in the α – naphthol – sulfuric acid reaction, the indole – sulfuric acid reaction, the phenol – sulfuric acid reaction, and the tryptophane – sulfuric acid reaction; containing 43.5 to 45.3% by weight of carbon; 5.7 to 6.7% by weight of hydrogen and the balance of oxygen and being free of nitrogen; the saccharide units of said polysaccharide being composed principally of D – glucose bonded together entirely by α – linkages and having a structure in which

$$\rightarrow {}^{4}G^{1} \rightarrow$$

is within the range of 3.5 to 8.5,

$$\rightarrow {}^{3}G^{1} \rightarrow$$

is less than 2,

$$\begin{array}{c}\rightarrow {}^{4}G^{1}_{6} \rightarrow \\ \uparrow\end{array}$$

is within the range of 0.5 to 2.0,

$$\begin{array}{c}\rightarrow {}^{4}G^{1}_{3} \rightarrow \\ \uparrow\end{array}$$

is within the range of 0.1 to 2.5 and

$$\begin{array}{c}\rightarrow {}^{3}G^{1}_{6} \rightarrow \\ \uparrow\end{array}$$

is less than 0.8 when the non – reducing endgroup ($G^1 \rightarrow$ ) of monosaccharide as determined in methylation – hydrolysis test according to Haworth's method is indexed as 1; the specific rotatory power $[\alpha]_D^{25}$ of said polysaccharide being + 70° to + 180°; said polysaccharide showing a specific absorption at 840 $cm^{-1}$ in its infrared absorption spectrum; showing absorptions at 3.7 ± 0.1, 3.8 ± 0.1, 5.0 ± 0.1, and 5.4 ± 0.1 ppm but not showing any absorptions in the range of 4.4 to 4.9 ppm in its nuclear magnetic resonance spectrum; and said polysaccharide being soluble in water but insoluble in pyridine, chloroform, and hexane. This polysaccharide is produced by a process comprising the steps of;
extracting mycelia, fruit bodies of a Basidiomycetes fungus selected from the group consisting of Coriolus versicolor (Fr.) Quél., Coriolus consors (Berk.) Imaz., Coriolus hirsutus (Fr.) Quél. and Coriolus pargamenus (Fr.) Pat. or mixtures thereof with an aqueous solvent selected from the group consisting of water, an aqueous dilute acid solution, an aqueous 0.005 to 2N solution of potassium – or sodium hydroxide, and an aqueous dilute solution of an organic solvent,
saturating the thus – obtained extract solution with ammonium sulfate after removing the low – molecular

weight substances with molecular weight of lower than 5,000 contained therein by ultrafiltration, reverse osmosis or a combination thereof,
collecting the resultant precipitate,
dissolving said precipitate in water,
desalting the thus-obtained solution of said precipitate,
passing the thus-desalted solution through a column packed with an ion exchanger, thereby absorbing and removing the nitrogenous substance contained therein,
concentrating the thus-obtained solution, and
drying the thus-obtained concentrate to obtain said polysaccharide.

JP-A-57-40,159 (1982) discloses a protein-bound polysaccharide possessing a molecular weight in the range of 5,000 to 300,000 as measured by the ultracentrifugal method; exhibiting positive color reactions characteristic of saccharides in $\alpha$-naphthol sulfuric acid reaction, indole sulfric acid reaction, and tryptophane sulfuric acid reaction; exhibiting positive color reactions characteristic of peptide bonds and amino acids in the Lowry-Folin method and the ninhydrin reaction after hydrochloric acid hydrolysis; possessing absorption regions of 0.9 ± 0.1 ppm, 1.2 ±0.1 ppm, 2.0 ± 0.1 ppm, 4.5 ± 0.1 ppm, 4.7 ± 0.1 ppm, 5.0 ± 0.1 ppm, and 5.4 ± 0.1 ppm and a broad absorption region of 3.0 to 4.4 ppm in the proton nuclear magnetic resonance spectrum; having a ratio of saccharide moiety to protein moiety in the range of 55/45 to 95/5 provided that the proton strength of the protein moiety falls in the range of 0.5 to 2.5 ppm and and the proton strength of the saccharide moiety is in the range of 2.5 to 6.0 ppm; having the saccharide moiety comprising $\beta$-D-glucan and $\alpha$-D-glucan, and a ratio of absorption intensity ($\alpha/\beta$) of the absorption attributed to the $\alpha$-glucan of 4.9 to 6.0 ppm to the absorption attributed to the $\beta$-glucan of 4.4 to 4.9 ppm in the range of 50/50 to 10/90; and having the protein moiety composed of aspartic acid, threonine, serine, glutamic acid, proline, glycine, alanine, cysteine, valine, methionine, isoleucine, leucine, tyrosine, tryptophane, phenyl alanine, lysine, histidine and arginine. A process is disclosed for producing the aforementioned protein-bound polysaccharide comprising preculturing a seed culture of a basidiomycetous fungus thereby inducing growth of a fungal lichen on the surface of a culture medium, homogenizing the fungal lichen with physiological saline solution thereby producing a seed culture for production culture, cultivating the seed culture by stationary or submerged culture thereby obtaining a mycelium, extracting the mycelium with hot water or an aqueous solvent such as dilute alkali solution, concentrating the extract after removal of residue therefrom, salting out the concentrated extract with ammonium sulfate or subjecting to ultrafiltration for removal of low molecular substances and production of a refined concentrate, saturating the resultant concentrate to 40% with ammonium sulfate, removing from the concentrate the precipitate, then saturating the resultant solution to 60% with ammonium sulfate, collecting from the solution the thus formed precipitate, desalting a solution of the separated precipitate by dialysis, subjecting the desalted solution to DEAE-cellulose column, and subsequently eluting the adsorbate with an aqueous 1 mol sodium chloride solution.

US-A-4 663 438 discloses a nucleic acid-containing glycoprotein, having a molecular weight of from 5,000 to 300,000 as determined by the ultracentrifugation method; the ratio of the weight of its protein moiety, as determined by Lowry-Folin,s method, to the weight of its saccharide moiety, as determined by the phenosulfuric acid method, being from 50:50 to 80:20; the saccharide moiety containing fucose, ribose, arabinose, xylose, mannose, galactose, glucose and glucosamine, and the total weight of said xylose, said mannose and said glucose being more than 85% by weight of the total weight of said saccharides; the protein moiety containing the amino acids aspartic acid, threonine, serine, glutamic acid, proline, glycine, alanine, cysteine, valine, methionine, cystathionine, isoleucine, leucine, tyrosine, phenylalanine, tryptophane, ornithine, lysine, histidine and arginine, and the total weight of said aspartic acid, threonine, serine, glutamic acid, glycine, alanine, phenylalanine, valine, leucine and isoleucine being more than 75% by weight of the total weight of all of said amino acids; the amino acid at its N-end being tyrosine, leucine or alanine; the amino acid sequence at its C-end being valine-phenylalanine-leucine, the terminal amino acid being leucine; its elementary composition being from 35.2 to 49.3% of C, from 4.8 to 8.0% of H, from 4.3 to 12.3% of N, from a trace amount to 2.5% of S, from a trace amount to 1.2% of P and the balance being O; the isoelectric point being from pH 2.5 to pH 5.0; the nucleic acid containing 0.01 to 0.50% by weight of uracil as a base of nucleic acid; and showing infrared absorption maxima at 3600-3200 $cm^{-1}$, 1530 $cm^{-1}$ and 1200-1000 $cm^{-1}$. The nucleic acid-containing glycoprotein is obtained by extracting fruit bodies, mycelia or cultured mycelia of a fungus belonging to Coriolus of Basidiomycetes with hot water or an aqueous 0.01 to 2.0N alkali solution at a temperature of 80 to 100°C for 1 to 8 hours, neutralizing the obtained extract, subjecting the neutralized extract to dialysis and/or ultrafiltration thereby removing a low molecular weight substance having a molecular weight of below 5,000 and fractionally collecting the fractions precipitating at a pH of 2.5 to 5.0 and ionic strength of 0.1 to 3.1$\mu$M at a temperature of 5 to 25°C.

JP-A-51-36,322 (1976) discloses a polysaccharide which, in the form of the hydrolyzate, exhibits positive color reactions in the Molisch reaction, the anthrone sulfuric acid reaction, the tryptophane sulfuric acid reaction, the cysteine sulfuric acid reaction, the aminoguanidine sulfuric acid reaction, the Chromotropic sulfuric acid reaction, the carbazol-cysteine sulfuric acid reaction, the Seliwanoff reaction, the Bial test, the aniline hydrochloric acid reaction, the Tollens reaction and the thioglycolic acid sulfuric acid reaction, and a weakly positive color reaction in the ninhydrin reaction. The polysaccharide possesses an anticancer activity. The process for producing the polysaccharide comprises artificially culturing a fungus of genus Coriolus thereby producing a mycelium and extracting the mycelium with water or an aqueous solvent containing a small amount of acid, base or organic solvent.

US-A-4 051 314 discloses a process for the production of a polysaccharide comprising forming a culture medium or a liquid extract of mycelium of a species of fungi belonging to the class Basidiomycetes and selected from the group consisting of Coriolus versicolor, Coriolus conchifer, Coriolus pargamenus, Coriolus hirsutus, Coriolus biformis, Coriolus consors and Coriolus pubescens, separating said polysaccharide from the free proteins, and other impurities contained in said culture medium or liquid extract and purifying said polysaccharide to produce a purified polysaccharide having an acute toxicity (LD50) in mice of more than 20 g/kg for oral administration and more than 100 mg/kg for subcutaneous injection; and

a polysaccharide having a molecular weight within the range of 1.0-1.9 x 100000, as determined by gel filtration; which, in the form of a hydrolyzate, gives positive ninhydrin, anisaldehyde, molisch, anthrone, tryptophane-sulfuric acid, chromotropic acid-sulfuric acid, carbazole-cysteine-sulfuric acid, aniline-hydrochloric acid, resorcinol-hydrochloric acid, tollens and thioglycol-sulfuric acid reactions but negative ferric chloride and fehling reactions. The polysaccharide is produced by a process comprising forming a culture medium or a liquid extract of mycelium of a species of fungi belonging to the class Basidiomycetes and selected from the group consisting of Coriolus versicolor, Coriolus conchifer, Coriolus pargamenus, Coriolus hirsutus, Coriolus biformis, Coriolus consors and Coriolus puberscens, and separating the polysaccharide from said culture medium or liquid extract.

JP-A-56-14274 discloses a method for producing a nitrogen-containing polysaccharide, comprising extracting a fungus belonging to Coriolus of Basidiomycetes with an aqueous 0.01N to 2N alkali solution and subjecting the resultant extract to ultrafiltration or reverse osmosis thereby removing from the extract low molecular substances of molecular weights not exceeding 5,000.

In recent years, various viral diseases such as B-type hepatitis, adult T-cell leukemia, and AIDS (Acquired Immunodeficiency Syndrome) have been subjects of scientific research.

Particularly, a series of acquired immunodeficiency syndromes popularly referred to as "AIDS" have been attracting interest as a serious disease fatal to the patients. It has been already established by research that contraction of this disease occurs when the human $T_4$ lymphocytic cells adsorb HIV (human immunodeficiency virus), one species of retrovirus. Propagation of the disease follows when the other lymphatic cells are infected by the virus. Generally, the viral diseases have been heretofore coped with by preventive inoculation of relevant vaccines. As a result, smallpox has been exterminated, and yellow fever and polimyelitis (infantile paralysis) have been attacked.

However, the vaccination is not effective in curing such diseases as AIDS which pose the problem of persistent infection or latent infection. In the light of nature of the pellicle of HIV, it is considered difficult to develop a vaccine which is effective in attacking the HIV. In the circumstances, the development of a safe anti-AIDS drug which manifests an outstanding effect in curing the disease is desired.

One of the present inventors has already found a number of protein-bound polysaccharides having an activity as biological response modifiers (BRM) of the immune system. Particularly, one species of protein-bound polysaccharide extracted from a fungus belonging to Coriolus of Basidiomycetes has already been sold as an anti-tumour drug under Trademark of "Krestin". This drug shows very low toxicity and can be administered for a long time without any fear of disturbing intestinal microbes. Further, it is an extremely safe substance because it has no adverse effect on the nature of variation or the reaction of allergy and, therefore, has no possibility of inducing deformation or allergy on a healthy person. The active protein-bound polysaccharides extracted from fungi belonging to Coriolus of Basidiomycetes are natural substances and are complicated compounds which are formed with numerous other protein-bound polysaccharides. Therefore, the active polysaccharides, which are effective in curing AIDS [obstructing the adsorption of HIV by human lymphatic cells and inhibiting the activity of RTxase (reverse transcriptase) which is an enzyme essential for the propagation of HIV] have not yet been fully elucidated.

Accordingly, the present invention provides a protein-bound polysaccharide

(i) possessing a molecular weight of from 50,000 to 3,000,000 as measured by gel permeation chromatography;

(ii) exhibiting a positive colour reaction characteristic in the $\alpha$-naphthol sulfuric acid reaction, the indole sulfuric acid reaction, the anthrone sulfuric acid reaction, the phenol sulfuric acid reaction, the tryptophane sulfuric acid reaction, the Lowry-Folin method and the ninhydrin reaction after hydrochloric acid hydrolysis;

(iii) in which the ratio of the weight of the protein moiety determined by the Lowry-Folin method to the weight of the saccharide moiety determined by the phenol sulfuric acid method is from 40:60 to 70:30;

(iv) showing solubility in water and insolubility in pyridine, chloroform, benzene, hexane and methanol;

(v) exhibiting a specific rotation, $[\alpha]_D^{25}$, of from $-10°$ to $30°$;

(vi) showing a characteristic absorption at 890 $cm^{-1}$ in the infra-red absorption spectrum;

(vii) containing the amino acids aspartic acid, threonine, serine, glutamic acid, glycine, alanine, valine and leucine in an amount of not less than 70% by weight of the total weight of the amino acids of the protein moiety thereof;

(viii) containing as saccharides glucose and mannose in an amount of not less than 75% by weight of the total weight of the saccharides of the saccharide moiety thereof, the ratio of glucose to mannose being of form 2:1 to 4:1;

(ix) containing no nucleic acid as measured by high speed liquid chromatography using ultra-violet light at 245 nm; and

(x) wherein the saccharide moiety is represented by the following structural formula (I):

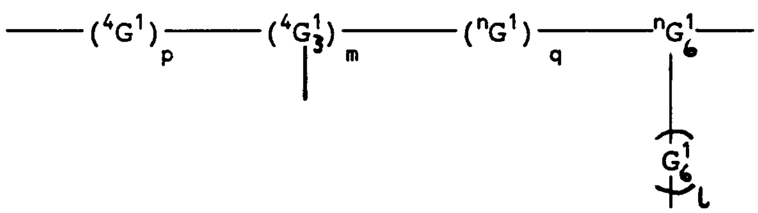

wherein G represents a monosaccharide, p is from 0 to 10, q is from 0 to 5, m from 0 to 2, n is 3 or 4, and 1 is 0 or 1.

The above protein-bound polysaccharide generally has an average molecular weight of from 110,000 to 300,000 as measured by gel permeation chromatography. The bonds between the saccharide moiety and the protein moiety are mainly via a glucosamine residue.

The above protein-bound polysaccharide is obtained by a process comprising culturing a fungus belonging to Coriolus of Basidiomycetes, extracting the mycelium or fruit bodies with water or an aqueous alkaline solution and isolating the said polysaccharide from the extract by salting out said extract with ammonium sulfate and subjecting the resulting solution to purification on an ion-exchange cellulose column. The fungus is suitably strain CM 101, CM 102 or CM 103 of Coriolus versicolor (Fr.) Quel.

The above process is preferably a process wherien a solution of the precipitate obtained by salting out the said extract with saturated ammonium sulfate is desalted and the said polysaccharide is obtained by : (1) salting out the resultant solution with ammonium sulfate of a saturation degree of 25%, desalting a solution of the obtained precipitate, introducing the desalted solution onto a DEAE-ion-exchange cellulose column, eluting the adsorbate with an aqueous sodium chloride solution and desalting the elute or (2) introducing the thus desalted solution onto a DEAE-ion-exchange cellulose column, eluting the adsorbate with an aqueous sodium chloride solution, desalting the elute, salting out the resultant solution with ammonium sulfate of a saturation degree of 25% and desalting a solution of the obtained precipitate.

The protein-bound polysaccharide is useful as an anti-viral agent, and is particularly useful as an anti-retroviral agent. Thus, the polysaccharide may be used in the treatment of AIDS.

This invention therefore includes a pharmaceutical composition comprising, as an active ingredient, a protein-bound polysaccharide as defined above and a pharmaceutically acceptable carrier.

Brief Description of the Drawings:

Fig. 1 is an infrared absorption spectrum of the substance obtained in Example 1 and Fig. 2 is an NMR spectrum of the same substance.

The protein-bound polysaccharide of the present invention is hereinafter referred to as the present substance.

(1) Preparation of the present substance

The present substance is obtained by culturing a fungus of Coriolus of Basidiomycetes, for example, Coriolus versicolor (Fr.) Quél., Coriolus hirsutus (Fr.) Quél., Coriolus pargamenus (Fr.) Pat. and Coriolus consors (Berk.) Imaz., thereby inducing growth of a mycelium or fruit body, extracting the mycelium or fruit body with hot water or an aqueous alkaline solution, salting out the resultant extract with saturated ammonium sulfate, after redissolving the resultant precipitate, desalting the thus obtained solution by dialysis, (i) salting out the resultant solution with ammonium sulfate equivalent to a saturation degree of 25%, desalting a solution of the resultant precipitate by dialysis, subjecting the resultant solution to DEAE-ion-exchange cellulose column thereby adsorbing a fraction thereon, then eluting the adsorbed fraction with an aqueous sodium chloride solution, and desalting the eluate by dialysis, or (ii) subjecting the resultant solution to DEAE-ion-exchange cellulose column, eluting the adsorbed fraction with an aqueous sodium chloride solution, desalting the eluate by dialysis, salting out the resultant solution with ammonium sulfate equivalent to a saturation degree of 25%, redissolving the resultant precipitate, and then desalting the thus obtained solution by dialysis.

(2) Physical and chemical properties

① Molecular weight

The molecular weight of the present substance is in the range of 50,000 to 3,000,000 and the average molecular weight thereof is in the range of 110,000 to 300,000 as measured by the gel permeation chromatography.

② Color reaction

When the present substance is dissolved in water for color reaction, the following results are obtained.

Table 1

| Color reaction | Color | Result |
|---|---|---|
| α-Naphthol sulfuric acid reaction (Molisch reaction) | Purple | Glycide |
| Indole sulfuric acid reaction | Brown | Glycide |
| Anthrone sulfuric acid reaction | Green | Glycide |
| Phenol Sulfuric acid reaction | Brown | Glycide |
| Tryptophane sulfuric acid reaction | Purple | Glycide |
| Lowry-Folin method | Blue | Peptide bond, tyrosine, tryptophane, and cysteine |
| Ninhydrin reaction after hydrochloric acid hydrolysis (6-N HCl, 110°C, 20 hrs) | Purplish blue | α-Amino acid |

From the foregoing results of the color reactions, it is clear that the present substance contains saccharides and proteins.

③ Solubility

The present substance is soluble in water and substantially insoluble in methanol, pyridine, chloroform, benzene and hexane.

④ pH value

A solution of 1 g of the present substance in 100 ml of water shows a pH value of 6.0 to 7.5, indicating that the substance is nearly neutral.

⑤ Specific rotation

The specific rotation (%), $[\alpha]_D^{25}$, calculated from the optical properties determined of an aqueous solution containing the present substance in a concentration of 0.10% is in the range of $-10°$ to $+30°$, implying that the present substance has β-glycan as the main component thereof.

⑥ Elementary analyses

By elementary analysis, the present substance is found to be composed of 5 to 10% of nitrogen, 35 to 50% of carbon and 5 to 7% of hydrogen.

⑦ The weight ratio of the content of the saccharide moiety of the present substance determined by the phenol sulfuric acid method to the content of the protein moiety of the present substance determined by

the Lowry-Folin method falls in the range of 60/40 to 30/70, preferably 54/46 to 35/65.

(3) Structural characteristic

The characteristic of the protein moiety and the characteristic of the saccharide moiety of the present substance are as follows.

① Characteristic of protein moiety

The protein moiety of the present substance is hydrolyzed and then analyzed with an amino acid analyzer in accordance with the conventional process. An amino acid composition is shown below in Table 2.

Table 2

| Amino acid | Weight (%) |
|---|---|
| Aspartic acid | 10 ~ 19 |
| Threonine | 4 ~ 10 |
| Serine | 3 ~ 11 |
| Glutamic acid | 10 ~ 18 |
| Glycine | 6 ~ 9 |
| Alanine | 6 ~ 13 |
| Valine | 5 ~ 11 |
| Leucine | 6 ~ 8 |
| Proline | Trace ~ 8 |
| Cystine | Trace |
| Methionine | Trace ~ 4 |
| Isoleucine | 3 ~ 5 |
| Tyrosine | Trace ~ 3 |
| Phenylalanine | 3 ~ 6 |
| Tryptophane | Trace ~ 2 |
| Lysine | 1 ~ 4 |
| Histidine | Trace ~ 2 |
| Arginine | 1 ~ 4 |
| (Ammonia) | 1 ~ 6 |

From the Table given above, it is clearly noted that the protein moiety as an active component of the present substance contains 18 amino acids and has acidic amino acids and neutral amino acids as major components and basic amino acids as minor component. It is further noted that not less than 70% of the total amount of the amino acids is composed of aspartic acid, threonine, serine, glutamic acid, glycine, alanine, valine, and leucine.

② Structure of saccharide moiety

The test of the saccharide moiety of the present substance for saccharide composition is carried out by subjecting 10 mg of a sample to methanolysis at 100°C for 16 hours with 3% methanol hydrochloride, then subjecting the product after methanolysis to trimethylsilyl treatment by the conventional process, and analyzing the thus obtained product by gas chromatography. The results of this test indicate that a weight ratio of glucose to mannose is in the range of 2 : 1 to 4 : 1, and principal component saccharides of the saccharide moiety are glucose and mannose, and not less than 75% of the saccharide moiety is composed of glucose and mannose. Such saccharides as galactose, xylose, fucose, and glucosamine are also contained therein.

The saccharide moiety of the present substance, for the purpose of confirming the D-L distinction of glucose which is one of the main component saccharides thereof, is hydrolyzed and the glucose crystals isolated from the hydrolyzate are assayed, and as a result the glucose crystals are found to possess a melting point of 143 to 145°C, thus showing no fall of melting point and identifying themselves to be D-glucose.

③ Bonding characteristic of component saccharides of the saccharide moiety

The bonding position of glycoside is determined as follows. The monosaccharides isolated from the saccharide moiety by the periodic acid oxidation method and the Smith degradation method are confirmed to possess the bonds, $G^1-$, $-^4G^1-$,

$$-{}^{4}G^{1}_{3}-,\quad -{}^{4}G^{1}_{6}-,$$

$-{}^{3}G^{1}-$,

$$-{}^{3}G^{1}_{6}-,$$

and $-{}^{6}G^{1}-$. The percentage of these bonds is determined by methylating hydrolysis in accordance with the Haworth method. The identification is performed as follows. The saccharides formed by the hydrolysis of the methylation product are identified as alditol acetate and methyl glycoside by gas chromatography. Further the individual hydrolyzates are identified by being isolated by column liquid chromatography and then directly crystallized or converted into crystalline derivatives. The molar ratios are calculated from the ratios of areas formed on the gas chromatograph of alditol acetate, based on

$$\rightarrow {}^{4}\underset{\uparrow}{G}^{1} \rightarrow \;+\; \rightarrow {}^{3}G^{1}_{\uparrow 6} = 1.$$

Table 3

| Hydrolyzate of methylated saccharide | Bond | Molar ratio |
|---|---|---|
| 2,3,6-Tri-O-methyl-G | $\rightarrow {}^{4}G^{1} \rightarrow$ | 0 ∿ 10 |
| 2,3,4-Tri-O-methyl-G | $\rightarrow {}^{6}G^{1} \rightarrow$ | 0 ∿ 1 |
| 2,3-Di-O-methyl-G | $\rightarrow {}^{4}G^{1}_{\uparrow 6} \rightarrow$ | 0 ∿ 1 |
| 2,6-Di-O-methyl-G | $\rightarrow {}^{4}G^{1}_{\uparrow 3} \rightarrow$ | 0 ∿ 2 |
| 2,4,6-Tri-O-methyl-G | $\rightarrow {}^{3}G^{1} \rightarrow$ | 0 ∿ 5 |
| 2,4-Di-O-methyl-G | $\rightarrow {}^{3}G^{1}_{\uparrow 6} \rightarrow$ | 0 ∿ 1 |

It is clearly shown from the table given above that the saccharide moiety of the present substance is formed mainly with the $\beta-1,4$ linkage. The saccharide moiety nevertheless contains the $\beta-1,3$ linkage. Thus, it is found to possess a structure highly abounding with branches.

The saccharide moiety, therefore, is concluded to possess a structure represented by the following general formula (I):

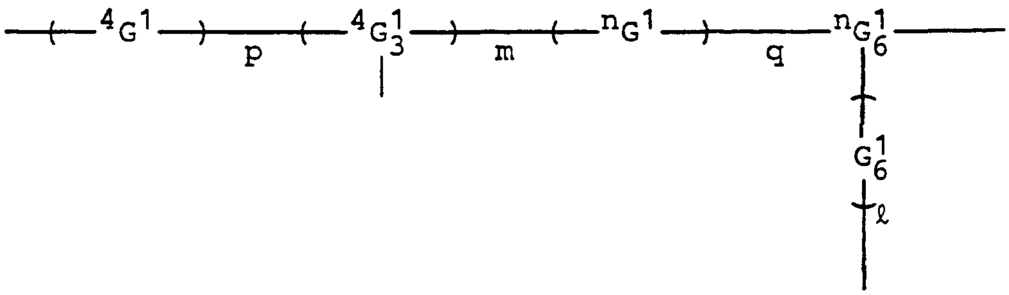

(wherein G represents a monosaccharide, p is in the range of 0 to 10, q is in the range of 0 to 5, m is in the range of 0 to 2, n is 3 or 4, and ℓ is in the range of 0 to 1).

The bonds between the saccharide moiety and the protein moiety of the present substance mainly contain those bonds which are formed via the medium of glucosamine. The structure of the present substance is specifically such that either of the hydroxyl groups bonded to the carbon atoms at the positions of C3, C4, and C6 of glucosamine is bonded to the saccharide moiety, and the hydroxyl group at the C1 position and the protein moiety are bonded to each other through the N-glucoside bond.

④ Infrared spectrum

The infrared absorption spectrum of the present substance obtained by the KBr tablet method is as shown in Fig. 1. The broad absorption region of 3,600 to 3,200 $cm^{-1}$ in Fig. 1 is presumed to originate from $\nu$OH's hydrogen bonded to varying degrees. This presumption is based on the observation that this absorption either diminishes or disappears when the hydroxyl groups of the saccharide moiety of the sample is O-methylated. The absorption of 1,700 to 1,600 $cm^{-1}$ is presumed to arise from the deformation vibration of $-NH_2$ and the absorption of 1,530 $cm^{-1}$ is presumed to arise from that of -NH respectively, and they are considered to originate from the protein moiety of the sample. The broad absorption of 1,200 to 1,000 $cm^{-1}$ is presumed to arise from the asymmetrical stretching vibration of the pyranose ring C-O-C bond in the saccharide moiety. While a specific absorption attributed to the $\beta$-configuration of glucose of the saccharide moiety is recognized at 890 $cm^{-1}$, virtually no specific absorption attributed to the $\alpha$-configuration is recognized at 840 $cm^{-1}$. The aforementioned infrared absorption spectrum is not recognized to reveal any significant error with respect to the present substance.

⑤ Proton nuclear magnetic resonance (NMR)

The NMR of the present substance is obtained at 100 MHz using heavy water as a solvent and sodium 2,2-dimethyl-2-silanopentane-3-sulfonate (D.S.S.) as an internal standard. In Fig. 2 which shows the spectrum consequently obtained, since the absorption at 4.5 ppm is known to be an absorption attributed to the $\beta$-(1-4) and $\beta$-(1-6) in the methine proton at the 1 position and the absorption at 4.7 ppm to be an absorption attributed to the $\beta$-(1-4) and $\beta$-(1-6) in the methine proton at the 1 position, the ratio of $\beta$-(1-4) and $\beta$-(1-6) to $\beta$-(1-3) can be determined. Since the structure of the present substance contains branches, precise elucidation of the structure must rely inevitably on the methylation method. The absorption at 5.0 ppm is attributed to the $\alpha$-(1 → 6) and absorption at 5.4 ppm is attributed to the $\alpha$-(1 → 4) and $\alpha$-(1 → 3).

Based on D.S.S., the present substance possesses absorption at 0.9 ± 0.1 ppm, 1.2 ± 0.1 ppm, 2.0 ± 0.1 ppm, 4.5 ± 0.1 ppm, and 4.7 ± 0.1 ppm, no absorption in the region of 4.9 to 6.0 ppm, and a broad absorption in the region of 3.0 to 4.4 ppm. The absorptions in the region of 0.5 to 2.5 ppm are attributed to the side-chain proton of the protein moiety and those in the zone of 2.5 to 4.7 ppm are attributed to the proton of the saccharide moiety.

As described above, the present substance is a novel $\beta$-glycopeptide obtained from a protein-bond polysaccharide derived from a fungus of Coriolus of Basidiomycetes, and containing no $\alpha$-glycan as determined by NMR and no nucleic acid as determined by high speed liquid chromatography using ultraviolet light at 254 nm.

(4) Acute toxicity

The present substance is highly safe for living organisms because it manifests very low toxicity and induces virtually no side effect.

The present substance is tested for acute toxicity by the following method.

Mice of the ICR-JCL strain 4 to 5 weeks in age and 21 to 24 g in weight, and rats of the Donryu rat of 4 to 5 weeks in age and 100 to 150 g in weight are used. The present substance is administered to the animals intravenously, subcutaneously, intraperitoneally, and orally. The animals to which the present substance is administered as diluted with physiological saline solution are kept under observation as to general symptom, fatality and body weight. At the end of the period of observation, the animals are sacrificed for anatomical examination.

As shown in Table 4, the present substance administered at the highest allowable dosage kills none of the animals. In fact, the present substance is extremely safe for living organisms so as to render the calculation of $LD_{50}$ value virtually impossible.

The test results establish that the present substance manifests very low acute toxicity and constitutes itself a safe drug.

Table 4

| Kind of animal | Manner of administration | $LD_{50}$ (mg/kg) | |
|---|---|---|---|
| | | Female | Male |
| Mouse | Intravenously | > 1300 | > 1300 |
| | Subcutaneously | > 5000 | > 5000 |
| | Intraperitoneally | > 5000 | > 5000 |
| | Orally | >20000 | > 20000 |
| Rat | Intravenously | > 600 | > 600 |
| | Subcutaneously | > 5000 | > 5000 |
| | Intraperitoneally | > 5000 | > 5000 |
| | Orally | >20000 | > 20000 |

(5) Test for antiviral activity

It has been known that generally a virus is reproduced through a path which comprises deposition of the virus on a target cell, injection of the nucleic acid of the virus into the cell, and subsequent integration of the nucleic acid into the genome of the cell. Particularly in the case of a retrovirus, the path for the reproduction requires to include a step in which RNA, the nucleic acid derived from the virus is transferred into DNA by the action of a RT xase before the nucleic acid is integrated into the genome of the cell.

The inventors have found that the present substance obstructs the deposition of HIV on the human lymphatic cell and the subsequent infection of disease and further obstructs the activity of RT xase. When the present substance is tested for the effect on HIV by a procedure which comprises treating HIV with the present substance used in a concentration of 50μg/ml (the present substance used in Examples 1 to 4) at 0°C for 2 hours, washing the treated HIV, adding the washed HIV to MT-4 cells, incubating the resultant mixture for 3 days, and subsequently taking count of the HIV antigen-positive cells, it is found that virtually all the HIV antigen-positive cells are no longer present, indicating that the present substance is highly effective in obstructing the deposition of HIV on the human lymphatic cells. When the present substance is tested for possible effect upon RT xase activity using the whole messenger RNA of rat liver as a mold, it is demonstrated that the present substance strongly inhibits RT xase activity even at a concentration of 5 μg/ml.

When the present substance is used as an anti-viral drug, it can be formulated in any desired form. The administration of the present substance may be effected in any desired manner.

When the present substance is prepared in the form of tablets, pellets, powder, or capsules for oral administration, the composition contained therein may suitably incorporate therein such additives as binder, enveloper, excipient, lubricant, disinegrator, and wetting agent as generally adopted pharmaceutically. When the present substance is used in the form of a solution for oral administration, it may be prepared as internal lotion, shake mixture, suspension, emulsion, or syrup. Otherwise, it may be prepared in the form of a dry product which is redissolved immediately prior to use. Such a liquid preparation may incorporate therein any of the additives and preserves in common use. When the present substance is prepared in the form of a solution for administration by injection, the composition of this solution may incorporate therein any of various additives such as stabilizer, buffer solution, preserving agent, and isotonic agent. It may be offered in unit-dose ampoules or in dispensing vials. The aforementioned composition may be in the form of aqueous solution, suspension, solution, or emulsion in oily or aqueous vehicle. The active component may be in the form of powder which, prior to actual use, is redissolved with a suitable vehicle such as sterilized water containing no pyrogenic substance.

The present substance is administered orally or parenterally to men and beasts. The oral administration embraces the form of administration which is effected by placing the preparation under the patient's tongue. The parenteral administration embraces the form of administration which is effected by subcutaneous, intramuscular, or intravenous injection or by instillation. The dosage of the antiviral agent of the present invention is affected by the discrimination between beasts and men as subjects of treatment, the age and individuality of a particular subject for treatment, and the condition of disease. There are times when the dosage must deviate from the range of to be specified below. Generally, for oral administration to a human subject, the daily dose is in the range of 0.1 to 1,000 mg, preferably 1 to 100 mg, per kg of body weight, to be taken as undivided or divided into two or three portions.

The present substance is effective in inhibiting viral contamination, particularly obstructing contamination of a retrovirus possessing a reverse transcriptase, and depressing AIDS which is caused by HIV infection.

Further, the present substance has a dual effect of obstructing the deposition of HIV on the human lymphatic cells and the subsequent infection and impeding RT xase activity.

The AZT which has already found utility as an anti-AIDS drug is observed to manifest a side effect of obstructing the mitosis of even normal cells. In contrast, the present substance manifests extremely low acute toxicity, shows virtually no side effect on normal cells, and proves to be a safe drug. It is a useful antiviral agent because it manifests an action of obstructing infection of virus, particularly a retrovirus. To be specific, the present substance is effective in preventing the infection of a virus, particularly the infection of a retrovirus, especially in depressing AIDS. Even when the present substance is used as an antiviral agent in combination with other agents such as AZT, the effect manifested by the present substance is not impaired. Thus, the combined use of the present substance with other medicine proves to be an effective measure.

Now, the present invention will be described more specifically below with reference to working examples. It should be noted, however, that this invention is not limited to these examples.

Examples 1 to 4:

Strains, CM 101 of Coriolus versicolor (Fr.) Quél [Fermentation Research Institute Deposit (FERM) P No. 2412 (corresponding to ATCC 20547); Examples 1 and 2], CM 102 of Coriolus versicolor (Fr.) Quél [FERM-P 2413 (corresponding to ATCC 20548); Example 3], and CM 103 of Coriolus versicolor (Fr.) Quél [FERM-P 2414 (corresponding to ATCC 20549); Example 4], were each inoculated to a 200-ml Erlenmeyer flask containing 30 ml of a culture medium using 5% of glucose, 0.2% of peptone, 0.3% of yeast extract, 0.1% of $KH_2PO_4$, and 0.01% of $MgSO_4.7H_2O$ and subjected to static culture at a temperature in the range of 25° to 27°C for 10 days. The fungal lichen consequently grown on the surface of culture medium was homogenized with physiological saline solution to prepare a seed culture. Then, in a 1.0-liter culture jar containing 200 ml of the same culture medium as described above, the aforementioned seed culture was inoculated and cultured therein at 25° to 27°C for 25 days to produce a mycelium of the relevant strain. The amount of the mycelium consequently obtained was 4 to 4.3 g/jar in the case of CM 101, 2.0 to 2.5 g/jar in the case of CM 102, and 2.7 to 3.2 g/jar in the case of CM 103.

Then, 100 g of mycelium of each strain was extracted with 3 liters of distilled water at 98°C for 3 hours while stirring. At the end of the extraction, the extract and the residue were separated from each other by centrifugal separator. This treatment of extraction to the thus separated residue was repeated. The extracts from the successive treatments were gathered and centrifuged for separation of the residue. Then, the resultant filtrate was concentrated.

The filtrate was treated with an aqueous solution of ammonium sulfate saturated to 100% to produce a precipitate. Then, the precipitate was redissolved in water and the resultant solution was desalted by dialysis using a cellulose membrane. The desalted solution was treated with an aqueous 25% ammonium sulfate solution. The precipitate consequently obtained was redissolved and desalted by dialysis. The desalted solution was subjected to adsorption using a DEAE-ion-exchange cellulose column. The adsorbed substances were eluted with an aqueous sodium chloride solution (1 mol). The eluate was desalted by ultrafiltration, then concentrated, and spray dried to obtain an objective substance of the present invention. The extraction method used in Examples 2 to 4 was performed in the same manner as the procedure described above, excepting that an aqueous 1/10-N caustic soda solution was used in the reextraction of residue instead of water and the pH value of the extracts was adjusted after completion of the extraction.

The infrared absorption spectrum of the substance obtained by the KBr tablet method as indicated in Table 5 was shown in Fig. 1. In this spectrum, the absorption of $\nu$OH in 2,600 to 3,200 $cm^{-1}$, the deformation vibration of $NH_2$ in 1,700 to 1,600 $cm^{-1}$, the deformation vibration of NH at 1,530 $cm^{-1}$, the broad absorption region in 1,200 to 1,000 $cm^{-1}$ attributed to the vibration of the pyranose ring, C-O-C bond of the saccaride moiety, and the specific absorption at 890 $cm^{-1}$ attributed to $\beta$-linkage of saccharide moiety were visible but the absorption at 840 $cm^{-1}$ attributed to $\alpha$-linkage was not clearly visible.

Since the infrared absorption spectra obtained of the four samples (Examples 1 to 4) were different very little, that of the sample of Example 1 is shown as a representative.

The NMR determination was performed by using D.S.S. as an internal standard and heavy water as a solvent. Since the NMR spectra obtained of the four samples (Examples 1 to 4) were different very little, that of the sample of Example 1 was shown as a representative.

By the gel permeation chromatography using a sepharose column, the molecular weight was found to fall in the range of 50,000 to 3,000,000 and the average molecular weight in the range of 110,000 to 300,000.

The analysis of amino acids was carried out by the steps of mixing 10 mg of a sample with 4 ml of an aqueous 6N hydrochloric acid solution, freezing the resultant mixture with dry ice acetone, then sealing a tube containing the frozen mixture under a vacuum, heating the contents of the sealed tube at 110°C for 24 hours to effect hydrolysis thereof, drying the resultant hydrolyzate, dissolving the dry hydrolyzate in 30 to 40 ml of a citrate buffer of pH 2.2, and analyzing the resultant solution with an amino acid analyzer.

The test for specific rotation was carried out by measuring the optical rotation ($\alpha$) of an aqueous solution containing a sample in a concentration of 0.10 % with the D line (589 $\mu$m) of sodium in a 5-cm cell and calculating the specific rotation, $[\alpha]_D^{25}$ , from the value of the optical rotation ($\alpha$).

The analysis of the saccharide for component mono-saccharides was carried out by placing 3 mg of a sample in a glass ampoule 5 mm in diameter, subjecting the sample to methanolysis with 1.0 ml of a 3% methanolic hydrogen chloride solution at 100°C for 16 hours, neutralizing the hydrochloric acid consequently formed with silver carbonate at room temperature, separating the product of neutralization by filtration, distilling the filtrate to dryness, dissolving the concentrated filtrate with 0.5 ml of dry pyridine, adding 0.2 ml of hexamethyl disilazane and 0.3 ml of trimethyl chlorosilane to the formed solution and allowing the resultant mixture to stand at rest at room temperature for 30 minutes to effect trimethylsilylation, thereafter dissolving the resultant product in chloroform, washing the resultant solution with cold water for removal of excess reagents, dehydrating the washed solution, evaporating the filtrate to dryness, dissolving the residue of the distillation in carbon tetrachloride, and analyzing the resultant solution by gas chromatography.

The mode of the bond of saccharides was determined by the Haworth method. To be more specific, this determination was carried out by dissolving 2 g of a sample in 10 ml of an aqueous 1-N NaOH solution, keeping the resultant solution vigorously stirred under a current of nitrogen gas at 40° to 50°C and, at the same time, adding 20 ml of dimethyl sulfuric acid and 40 ml of an aqueous 30% sodium hydroxide solution dropwise thereto over a period of several hours, allowing the resultant mixture to stand at rest overnight, treating the resultant solution with the same amount of methylation reagent, neutralizing the resultant reaction solution, dialyzing the product of neutralization with running water, vacuum-concentrating the dialyzate, then repeating the aforementioned methylation three times, again neutralizing and dialyzing the product of methylation with running water, then distillating the dialyzate to dryness under a vacuum, dissolving the residue of the distillation in 20 ml of a chloroform-methanol (10 : 1) mixture, adding a petroleum ether-ether (1 : 1) mixture to the resultant solution for precipitation of the methylation product, then hydrolyzing about 20 mg of the methylation product with an aqueous 1-N sulfuric acid solution at 100°C for 16 hours, and introducing the hydrolyzate into alditol acetate by the conventional method to

obtain a gas chromatogram. The molar ratios of the component monosaccharides were found from the peak areas on the gas chromatogram.

The degradated products mentioned above were identified on respective gas chromatograms using standard products. Then, hydrolyzates were isolated mutually by column liquid chromatography and were identified as directly crystallized or converted into crystalline derivatives.

Further, the color reactions, the elementary analysis, the acute toxicity ($LD_{50}$), the pH value, and the solubility were invariably measured by the conventional methods. The measurements whose procedures have been specifically described above were carried out by following these procedures.

By high speed liquid chromatography using ultraviolet light at 254 nm, nucleic acid of the four samples (Examples I – 4) was not found.

The properties and the structural characteristics of the present substance determined as described above are collectively shown in the accompanying tables.

The degree with which the present substance obstructs the reverse transcriptase activity specifically manifested by the retrovirus was determined by the following method.

In 10 ml of sterilized distilled water, 100 μg of a freeze dried sample of the present substance was dissolved (concentration 10 μg/ml).

An Eppendorf tube having an inner volume of 1.5 ml was charged with 1 μl of 20 mM D.T.T. (dithiothreitol; Sigma Corp), 5 μl of an enzyme reaction solution 5 times as high in concentration (250 mM Tris-HCl (pH 8.3)-250 mM KCl-40 mM $MgCl_2$), 1 μl of 3d NTP solution (1 mM dATP-1mM dGTp-1 mM dTTp; Sigma Corp), 1 μl of messenger-RNA (taken from normal rat liver: 1 μg/μl), 0.5 μl of RNase Inhibitor (16 units/μl; Takara Shuzo K. K.), and 1 μl of [ $\alpha$-$^{32}$P] dCTp (about 800 ci/m.mol, 10 μCi/μl; Amersham Japan K. K.) and was placed in a water bath at 37°C.

After 5 minutes' standing of the reaction tube in the bath, 12.5 μl of the present substance prepared in advance in a concentration of 10 μg/ml was added to the reaction tube and 1 μl of RT xase (derived from Rous Associated virus, 7 units/μl; Takara Shuzo K.K.) was further added thereto. The reaction solution was diluted to a final volume of 25 μl and kept at 37°C to induce reaction.

After the reaction continued for one hour, 5 μl of the reaction solution was allowed to permeate a DEAE paper 2 cm x 2 cm (Toyo Roshi K.K.). The wet paper was air dried. The dry filter paper was shaken as immersed in 10 ml per paper of an aqueous 0.5M $Na_2HPO_4$ solution to wash off the portion of [$\alpha$-$^{32}$P] dCTp which had escaped being used in the DNA synthesis. This procedure was performed five times at intervals of 5 minutes.

Thereafter, the aforementioned DEAE filter paper was placed in a glass vial containing 10 ml of liquid scintillation cocktail (Amersham Japan K.K.) and a radio-activity for one minute was counted by a scintillation counter (Aroka K.K.).

The ratio of inhibition of RTxase activity (%) was calculated on the following formula.

$$\text{Ratio of inhibition of RTxase activity (\%)} = \frac{C_O - C_S}{C_O} \times 100$$

(wherein $C_O$ represents the radioactivity in the absence of the present substance and $C_S$ for the radioactivity in the presence of the present substance

The results are shown in Table 5.

The inhibition of the deposition of HIV (AIDS virus) on human lymphocyte effected by the present substance was tested by the following method (The whole procedure was carried out under sterilized conditions).

A test tube containing 1 ml of a HIV suspension and 1 ml of a solution of the substance (100 μg/ml) was left standing at rest in ice. After the test tube stood in the ice for two hours, 1 ml of the virus suspension was left to be adsorbed on a cell strain derived from human lymphocyte, MT-4 [Jpn. J. Cancer Res. (Gann). 28, 219-229 (1982)] at an infection degree (M.O.I.) ≒ 2. The resultant mixture was centrifuged (2,000 rpm for 10 minutes). The supernatant was discarded and the sedimentod MT-4 cells were floated in RPMI 1640 (Gibco Laboratories, NY) containing 20% FCS in a cell concentration of 2 x $10^5$/ml.

On a 96-hole plate, the aforementioned MT-4 cell suspension was dispensed in a unit volume of 100 μl and incubated under the conditions of 5% $CO_2$ and 37°C. On the third day of the incubation, the sample suspensions were analyzed by the indirect fluorescent antibody method to determine the HIV infectious

deposition and the non-deposited cell.

To be more specific, the MT-4 cells were solidified by treatment with methanol and were left reacting with the anti-serum of a HIV infected patient at 37°C. After 30 minutes of the reaction, the cells were washed with PBS and then left reacting at 37°C with fluoresceine isothiocyanate-associated rabbit antihuman IgG (immune globulin).

Under a fluorescent microscope, 500 MT-4 cells were observed to take count of fluorescent positive cells as HIV-adsorbed cells and fluorescent negative cells as HIV-nonadsorbed cells. The ratio of inhibition of HIV deposition (%) was calculated based on the following formula using the results of the microscopic observation.

$$\text{Ratio of inhibition of HIV deposition (\%)} = \frac{(\text{Number of HIV-nonadsorbed cells}) \times 100}{(\text{Number of HIV-adsorbed cells}) + (\text{Number of HIV-nonadsorbed cells})}$$

The results are shown in Table 5.

Comparative Experiment 1:

Krestin was tested for ratio of inhibition of RTxase activity and ratio of inhibition of HIV deposition under the same conditions as in Examples 1 to 4. The ratios consequently found were both below 10%.

Example 5:

Capsules were obtained by packing hard capsules, No. 0, with 330 mg of the substance obtained in Example 1, with a pressure type automatic packing machine.

Table 5

| Example No. | 1 | 2 | 3 | 4 |
|---|---|---|---|---|
| Extraction method | Hot water | Aqueous alkaline solution | Aqueous alkaline solution | Aqueous alkaline solution |
| Molecular weight (x $10^4$) | 5 - 200 | 5 - 100 | 5 - 100 | 5 - 100 |
| Average molecular weight (x $10^4$) | 18 | 12 | 13 | 15 |
| Color reaction (saccharide) α-Naphthol sulfuric acid reaction | Purple | Purple | Purple | Purple |
| Indole sulfuric acid reaction | Brown | Brown | Brown | Brown |
| Anthrone sulfuric acid reaction | Green | Green | Green | Green |
| Phenol sulfuric acid reaction | Brown | Brown | Brown | Brown |
| Tryptophane sulfuric acid reaction | Purple | Purple | Purple | Purple |
| Coloration reaction (protein) Lowry-Folin method | Blue | Blue | Blue | Blue |
| Ninhydrin reaction (6N-HCl, 20 hr) after hydrochloric acid hydrolysis | Purplish blue | Purplish blue | Purplish blue | Purplish blue |

| Example No. | 1 | 2 | 3 | 4 |
|---|---|---|---|---|
| Specific rotation $[\alpha]_D^{25}$ (°) | -8 | +27 | +13 | +2 |
| pH | 6.6 | 7.2 | 7.4 | 7.0 |
| Absorption position (ppm) in NMR spectrum | | | | |
| 0.9 ± 0.1 | Yes | Yes | Yes | Yes |
| 1.2 ± 0.1 | Yes | Yes | Yes | Yes |
| 2.0 ± 0.1 | Yes | Yes | Yes | Yes |
| 4.5 ± 0.1 | Yes | Yes | Yes | Yes |
| 4.7 ± 0.1 | Yes | Yes | Yes | Yes |
| 5.0 ± 0.1 | No | No | No | No |
| 5.4 ± 0.4 | No | No | No | No |
| 3.0 ∿ 4.4 | Yes | Yes | Yes | Yes |
| Infrared absorption spectrum | | | | |
| 3600 - 3200 $cm^{-1}$ | Yes | Yes | Yes | Yes |
| 1600 $cm^{-1}$ | Yes | Yes | Yes | Yes |
| 1530 $cm^{-1}$ | Yes | Yes | Yes | Yes |
| 1200 - 1000 $cm^{-1}$ | Yes | Yes | Yes | Yes |
| 890 $cm^{-1}$ | Yes | Yes | Yes | Yes |
| 840 $cm^{-1}$ | No | No | No | No |

| Example No. | 1 | 2 | 3 | 4 |
|---|---|---|---|---|
| Amount of saccharide (% by weight) | 52.5 | 42.7 | 48.0 | 51.0 |
| Protein (% by weight) | 47.5 | 57.3 | 52.0 | 49.0 |
| Analysis of saccharides (mode of linkage) | | | | |
| $\rightarrow {}^{1}G^{4} \rightarrow$ | 10 | 4 | 2 | 4 |
| $\rightarrow {}^{1}G^{3} \rightarrow$ | 0.5 | 1.5 | trace | 2.0 |
| $\rightarrow {}^{1}G^{4}_{\uparrow 6} \rightarrow$ | 0.7 | 1.0 | 0.7 | 0.6 |
| $\rightarrow {}^{1}G^{6} \rightarrow$ | 0.9 | 0.7 | 0.4 | 0.8 |
| $\rightarrow {}^{1}G^{4}_{\uparrow 3} \rightarrow$ | 1.0 | 0.8 | 0.6 | 0.9 |
| $\rightarrow {}^{1}G^{3}_{\uparrow 6} \rightarrow$ | 0.3 | trace | 0.3 | 0.4 |
| Saccharides | | | | |
| Glucose | 71.3 | 60.7 | 69.0 | 60.5 |
| Mannose | 18.8 | 24.3 | 23.0 | 27.5 |
| Galactose | 3.8 | 4.8 | 3.8 | 5.4 |
| Xylose | 2.0 | 5.0 | 2.0 | 3.5 |
| Fucose | 3.2 | 4.0 | 1.1 | 1.5 |
| Glucosamine | 0.9 | 1.2 | 1.1 | 1.6 |

| Example No. | 1 | 2 | 3 | 4 |
|---|---|---|---|---|
| Ratio of component monosaccharides | | | | |
| Glucose/mannose | 3.8 | 2.5 | 3.0 | 2.2 |
| Nitrogen content (%) | 7.1 | 9.2 | 8.5 | 7.5 |
| Carbon content (%) | 45.1 | 46.2 | 39.1 | 41.4 |
| Hydrogen content (%) | 5.6 | 6.5 | 5.9 | 6.0 |
| Amino acid | | | | |
| Aspartic acid | 14.0 | 15.5 | 14.9 | 15.5 |
| Threonine | 8.0 | 7.0 | 8.4 | 7.0 |
| Serine | 7.6 | 6.5 | 9.7 | 7.8 |
| Glutamic acid | 12.7 | 16.8 | 15.5 | 16.9 |
| Proline | trace | 2.0 | trace | 1.0 |
| Glycine | 7.3 | 7.1 | 8.5 | 7.7 |
| Alanine | 10.0 | 10.3 | 10.4 | 11.0 |
| Cysteine | trace | trace | trace | trace |
| Valine | 8.5 | 6.9 | 7.8 | 6.0 |
| Methionine | 2.3 | 1.6 | 1.6 | 1.9 |
| Isoleucine | 4.8 | 4.1 | 3.1 | 4.2 |
| Leucine | 6.5 | 6.9 | 7.0 | 6.6 |
| Tyrosin | 0.3 | 1.1 | trace | 1.5 |
| Phenylalanine | 4.4 | 3.6 | 3.3 | 3.0 |

| Example No. | 1 | 2 | 3 | 4 |
|---|---|---|---|---|
| Tryptophane | 1.7 | 1.0 | 2.0 | trace |
| Lysine | 3.0 | 2.2 | 2.0 | 2.8 |
| Histidine | 1.8 | 1.4 | trace | 1.2 |
| Alginine | 2.7 | 3.0 | 2.4 | 2.2 |
| (Ammonia) | 4.4 | 3.0 | 3.4 | 3.7 |
| Total of aspartic acid, threonine, serine, glutamic acid, glycine, alanine, valine, and leucine | 74.6 | 77.0 | 82.2 | 78.5 |
| Acute toxicity for mouse ($LD_{50}$, mg/kg) | | | | |
| Intravenous, male | 1300< | 1300< | 1300< | 1300< |
| female | 1300< | 1300< | 1300< | 1300< |
| Subcutaneous, male | 5000< | 5000< | 5000< | 5000< |
| female | 5000< | 5000< | 5000< | 5000< |
| Intraperitoneal, male | 5000< | 5000< | 5000< | 5000< |
| female | 5000< | 5000< | 5000< | 5000< |
| Oral, male | 20000< | 20000< | 20000< | 20000< |
| female | 20000< | 20000< | 20000< | 20000< |

| Example No. | 1 | 2 | 3 | 4 |
|---|---|---|---|---|
| Acute toxicity for rat ($LD_{50}$, mg/kg) | | | | |
| Intravenous, male | 600< | 600< | 600< | 600< |
| female | 600< | 600< | 600< | 600< |
| Subcutaneous, male | 5000< | 5000< | 5000< | 5000< |
| female | 5000< | 5000< | 5000< | 5000< |
| Intraperitoneal, male | 5000< | 5000< | 5000< | 5000< |
| female | 5000< | 5000< | 5000< | 5000< |
| Oral, male | 20000< | 20000< | 20000< | 20000< |
| female | 20000< | 20000< | 20000< | 20000< |
| Ratio of inhibition of RTxase activity* | +++ | +++ | +++ | +++ |
| Ratio of inhibition of HIV deposition * | +++ | +++ | +++ | +++ |

* ±: <10%, +: 11 - 30%, ++: 31 - 70%, +++: >71%

## Claims

**1.** A protein-bound polysaccharide

(i) possessing a molecular weight of from 50,000 to 3,000,000 as measured by gel permeation chromatography;

(ii) exhibiting a positive color reaction characteristic in the $\alpha$-naphthol sulfuric acid reaction, the indole sulfuric acid reaction, the anthrone sulfuric acid reaction, the phenol sulfuric acid reaction, the tryptophane sulfuric acid reaction, the Lowry-Folin method and the ninhydrin reaction after hydrochloric acid hydrolysis;

(iii) in which the ratio of the weight of the protein moiety determined by the Lowry-Folin method to the weight of the saccharide moiety determined by the phenol sulfuric acid method is from 40:60 to 70:30;

(iv) showing solubility in water and insolubility in pyridine, chloroform, benzene, hexane and methanol;

(v) exhibiting a specific rotation, $[\alpha]_D^{25}$, of from $-10°$ to $30°$;

(vi) showing a characteristic absorption at 890 $cm^{-1}$ in the infra-red absorption spectrum;

(vii) containing the amino acids aspartic acid, threonine, serine, glutamic acid, glycine, alanine, valine and leucine in an amount of not less than 70% by weight of the total weight of the amino acids of the protein moiety thereof;

(viii) containing as saccharides glucose and mannose in an amount of not less than 75 % by weight of the total weight of the saccharides of the saccharide moiety thereof, the ratio of glucose to mannose being of from 2:1 to 4:1;

(ix) containing no nucleic acid as measured by high speed liquid chromatography using ultra-violet light at 245 nm; and

(x) wherein the saccharide moiety is represented by the following structural formula (I):

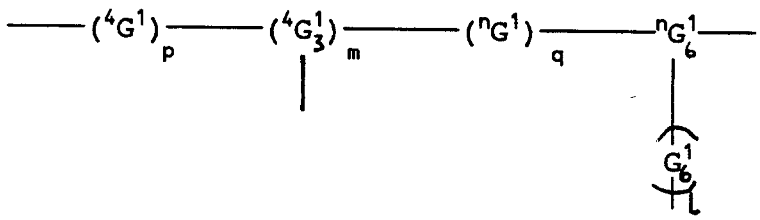

wherein G represents a monosaccharide, p is from 0 to 10, q is from 0 to 5, m from 0 to 2, n is 3 or 4, and 1 is 0 or 1.

**2.** A polysaccharide according to claim 1, which possesses an average molecular weight of from 110,000 to 300,000 as measured by gel permeation chromatography.

**3.** A polysaccharide according to claim 1 or 2, wherein the saccharide moiety is bonded to the protein moiety via a glucosamine residue.

**4.** A polysaccharide according to any one of the preceding claims, for use as an antiretroviral agent.

**5.** A polysaccharide according to claim 4 for use in the treatment of AIDS.

**6.** A process for the preparation of a polysaccharide as defined in claim 1, which process comprises culturing a fungus belonging to Coriolus of Basidiomycetes, extracting the mycelium of fruit bodies with water or an aqueous alkaline solution and isolating the said polysaccharide from the extract by salting out said extract with ammonium sulfate and subjecting the resulting solution to purification on an ion-exchange cellulose column.

**7.** A process according to claim 6, wherein the fungus is strain CM 101, CM 102 or CM 103 of Coriolus versicolor (Fr.) Quel.

**8.** A process according to claim 6 or 7, wherein a solution of the precipitate obtained by salting out the said extract with saturated ammonium sulfate is desalted and the said polysaccharide is obtained by : (1) salting out the resultant solution with ammonium sulfate of a saturation degree of 25%, desalting a solution of the obtained precipitate, introducing the desalted solution onto a DEAE-ion-exchange

cellulose column, eluting the adsorbate with an aqueous sodium chloride solution and desalting the elute or (2) introducing the thus desalted solution onto a DEAE-ion-exchange cellulose column, eluting the adsorbate with an aqueous sodium chloride solution, desalting the elute, salting out the resultant solution with ammonium sulfate of a saturation degree of 25% and desalting a solution of the obtained precipitate.

**9.** A pharmaceutical composition comprising, as an active ingredient, a protein-bound polysaccharide as defined in claim 1 and a pharmaceutically acceptable carrier.

## Patentansprüche

**1.** Proteingebundenes Polysaccharid,

(i) das ein mittels Gelpermeationschromatographie bestimmtes Molekulargewicht von 50 000 bis 3 000 000 besitzt;

(ii) das eine charakteristische positive Farbreaktion in der $\alpha$-Naphthol-Schwefelsäure-Reaktion, in der Indol-Schwefelsäure-Reaktion, in der Anthron-Schwefelsäure-Reaktion, in der Phenol-Schwefelsäure-Reaktion, in der Tryptophan-Schwefelsäure-Reaktion, bei der Lowry-Folin-Methode und, nach Hydrolyse mit Salzsäure, in der Ninhydrin-Reaktion zeigt;

(iii) bei dem das Verhältnis des Gewichts des Proteinanteils, das nach der Lowry-Folin-Methode bestimmt wurde, zum Gewicht des Polysaccharidanteils, das nach der Phenol-Schwefelsäure-Methode bestimmt wurde, von 40:60 bis 70:30 beträgt;

(iv) das eine Löslichkeit in Wasser zeigt und unlöslich in Pyridin, Chloroform, Benzol, Hexan und Methanol ist;

(v) das eine spezifische Drehung $[\alpha]_D^{25}$ von $-10°$ bis $+30°$ zeigt;

(vi) das im Infrarotabsorptionsspektrum eine charakteristische Absorption bei 890 $cm^{-1}$ zeigt;

(vii) das die Aminosäuren Asparaginsäure, Threonin, Serin, Glutaminsäure, Glycin, Alanin, Valin und Leucin in einer Menge von nicht weniger als 70 Gew.-%, bezogen auf das Gesamtgewicht der Aminosäuren im Proteinanteil, aufweist;

(viii) das als Saccharide Glucose und Mannose in einer Menge von nicht weniger als 75 Gew.-%, bezogen auf das Gesamtgewicht der Saccharide in dem Saccharidanteil, aufweist, wobei das Verhältnis von Glucose zu Mannose 2:1 bis 4:1 beträgt;

(ix) das keine Nucleinsäuren enthält, wobei die Messung mittels Hochgeschwindigkeits-Flüssigchromatographie unter Verwendung von ultraviolettem Licht bei 245 nm erfolgt; und

(x) wobei der Saccharidanteil durch die folgende Strukturformel (I) dargestellt wird:

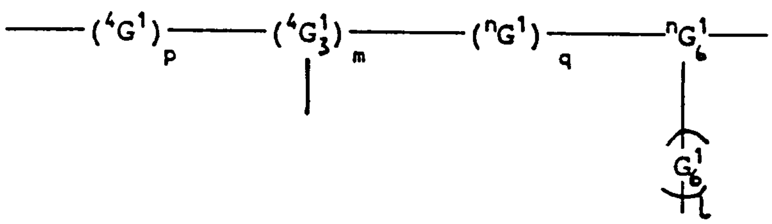

wobei G ein Monosaccharid darstellt, p einen Wert von 0 bis 10, q einen Wert von 0 bis 5, m einen Wert von 0 bis 2, n einen Wert von 3 oder 4 und 1 einen Wert von 0 oder 1 besitzt.

**2.** Polysaccharid nach Anspruch 1, das ein mittels Gelpermeationschromatographie gemessenes mittleres Molekulargewicht von 110 000 bis 300 000 besitzt.

**3.** Polysaccharid nach Anspruch 1 oder 2, wobei der Saccharidanteil über einen Glucosaminrest an den Proteinanteil gebunden ist.

**4.** Polysaccharid nach einem der vorstehenden Ansprüche zur Verwendung als antiretrovirales Mittel.

**5.** Polysaccharid nach Anspruch 4 zur Verwendung bei der Behandlung von AIDS.

6. Verfahren zur Herstellung eines Polysaccharids, wie es in Anspruch 1 definiert ist, wobei das Verfahren das Kultivieren eines Pilzes der Bezeichnung Coriolus der Basidiomycetes, das Extrahieren des Myzels und/oder der Fruchtkörper mit Wasser oder einer wäßrigen alkalischen Lösung und das Isolieren des Polysaccharids aus dem Extrakt durch Aussalzen des Extrakts mit Ammoniumsulfat und Unterwerfen der erhaltenen Lösung einer Reinigung über eine Ionenaustausch – Cellulose – Säule, umfaßt.

7. Verfahren nach Anspruch 6, wobei es sich bei dem Pilz um den Stamm CM 101, CM 102 oder CM 103 von Coriolus versicolor (Fr.) Quél. handelt.

8. Verfahren nach Anspruch 6 oder 7, bei dem eine Lösung des Niederschlags, der durch Aussalzen des Extrakts mit gesättigtem Ammoniumsulfat erhalten wurde, entsalzt wird, und bei dem das Polysaccharid durch folgende Stufen erhalten wird: (1) Aussalzen der erhaltenen Lösung mit Ammoniumsulfat bei einem Sättigungsgrad von 25 %, Entsalzen einer Lösung des erhaltenen Niederschlages, Aufbringen der entsalzten Lösung auf eine DEAE – Ionenaustausch – Cellulose – Säule, Eluieren des Adsorbats mit einer wäßrigen Natriumchloridlösung und Entsalzen des Eluats oder (2) Aufbringen der auf diese Weise entsalzten Lösung auf eine DEAE – Ionenaustausch – Cellulose – Säule, Eluieren des Adsorbats mit einer wäßrigen Natriumchloridlösung, Entsalzen des Eluats, Aussalzen der erhaltenen Lösung mit Ammoniumsulfat bei einem Sättigungsgrad von 25 % und Entsalzen einer Lösung des erhaltenen Niederschlages.

9. Pharmazeutische Zusammensetzung, die als aktiven Bestandteil ein proteingebundenes Polysaccharid, wie es in Anspruch 1 definiert ist, und einen pharmazeutisch verträglichen Träger umfaßt.

**Revendications**

1. Polysaccharide lié à une protéine

(i) d'un poids moléculaire de 50 x $10^3$ à 3 x $10^6$ mesuré au moyen d'une chromatographie par gel – filtration;

(ii) qui montre une réaction colorée positive caractéristique avec l'$\alpha$ – naphtol – acide sulfurique, l'indole – acide sulfurique, l'anthrone – acide sulfurique, le phénol – acide sulfurique, le tryptophane – acide sulfurique, avec la méthode de Lowry – Folin et lors du test à la ninhydrine après hydrolyse par l'acide chlorhydrique;

(iii) dans lequel le rapport, en poids, de la partie protéique, déterminée par la méthode de Lowry – Foline sur la partie saccharidique déterminée par la méthode phénol – acide sulfurique, est de 40:60 à 70:30;

(iv) soluble dans l'eau et insoluble dans la pyridine, le chloroforme, le benzène, l'hexane et le méthanol;

(v) qui montre une rotation spécifique, $[\alpha]^{25}{}_D$, dans un intervalle de $-10°$ à $30°$;

(vi) dont le spectre d'absorption infrarouge présente une absorption caractéristique à 890 $cm^{-1}$.

(vii) qui contient les acides aminés suivants: acide aspartique, thréonine, sérine, acide glutamique, glycine, alanine, valine et leucine en quantité au moins égale à 70%, en poids, de la totalité des acides aminés de la partie protéique;

(viii) qui contient, comme saccharides, du glucose et du mannose en quantité au moins égale à 75% en poids de la totalité des saccharides de la partie saccharidique, le rapport glucose sur mannose étant de 2:1 à 4:1;

(ix) dont la chromatographie liquide à grande vitesse en utilisant une lumière ultraviolette à 245 nm ne permet de déceler aucun acide nucléique et

(x) dans lequel la partie saccharidique est représentée par la formule structurale suivante (I):

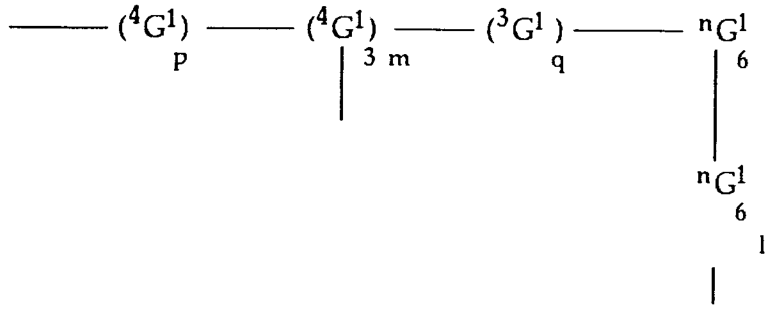

dans laquelle G représente un monosaccharide, p est de 0 à 10, q est de 0 à 5, m est de 0 à 2, n est 3 ou 4 et l est 0 ou 1.

**2.** Polysaccharide selon la revendication 1 qui possède un poids moléculaire moyen mesuré en chromatographie par gel-filtration de 110.000 à 300.000.

**3.** Polysaccharide selon la revendication 1 ou 2 dans lequel la partie saccharidique est liée à la partie protéique via un résidu glucosamine.

**4.** Polysaccharide selon l'une des revendications précédentes destiné à être utilisé en tant qu'agent antirétroviral.

**5.** Polysaccharide selon la revendication 4 utilisé dans le traitement du SIDA.

**6.** Procédé pour la préparation d'un polysaccharide tel que défini à la revendication 1, lequel procédé comprend la culture d'un champignon appartenant au genre Coriolus de la classe des Basidiomycètes, l'extraction du mycélium ou corps fructifère avec de l'eau ou avec une solution aqueuse alcaline et l'isolement dudit polysaccharide à partir de l'extrait en précipitant ledit extrait avec du sulfate d'ammonium et la purification de la solution ainsi obtenue sur une colonne de cellulose échangeuse d'ions.

**7.** Procédé selon la revendication 6, dans lequel le champignon appartient à souche CM 101, CM 102 ou CM 103 de Coriolus versicolor (Fr.) Quel..

**8.** Procédé selon la revendication 6 ou 7, dans lequel une solution du précipité obtenu par précipitation avec du sulfate d'ammonium saturé est dessalée et ledit polysaccharide est obtenu par: (1) précipitation de la solution ainsi obtenue avec une quantité de sulfate d'ammonium correspondant à un degré de saturation de 25%, le dessalage d'une solution du précipité ainsi obtenu, le passage de la solution ainsi dessalée sur une. colonne échangeuse d'ions de DEAE-cellulose, l'élution de la fraction adsorbée avec une solution aqueuse de chlorure de sodium et le dessalage de l'éluat ou (2) le passage de la solution ainsi dessalée sur une colonne échangeuse d'ions de DEAE-cellulose, l'élution de la fraction adsorbée avec une solution aqueuse de chlorure de sodium, le dessalage de l'éluat, la précipitation sélective de la solution ainsi obtenue avec une quantité de sulfate d'ammonium correspondant à un degré de saturation de 25% et le dessalage d'une solution du précipité obtenu.

**9.** Composition pharmaceutique comprenant comme principe actif un polysaccharide lié à une protéine comme défini à la revendication 1 et un support acceptable d'un point de vue pharmaceutique.

Fig. 1

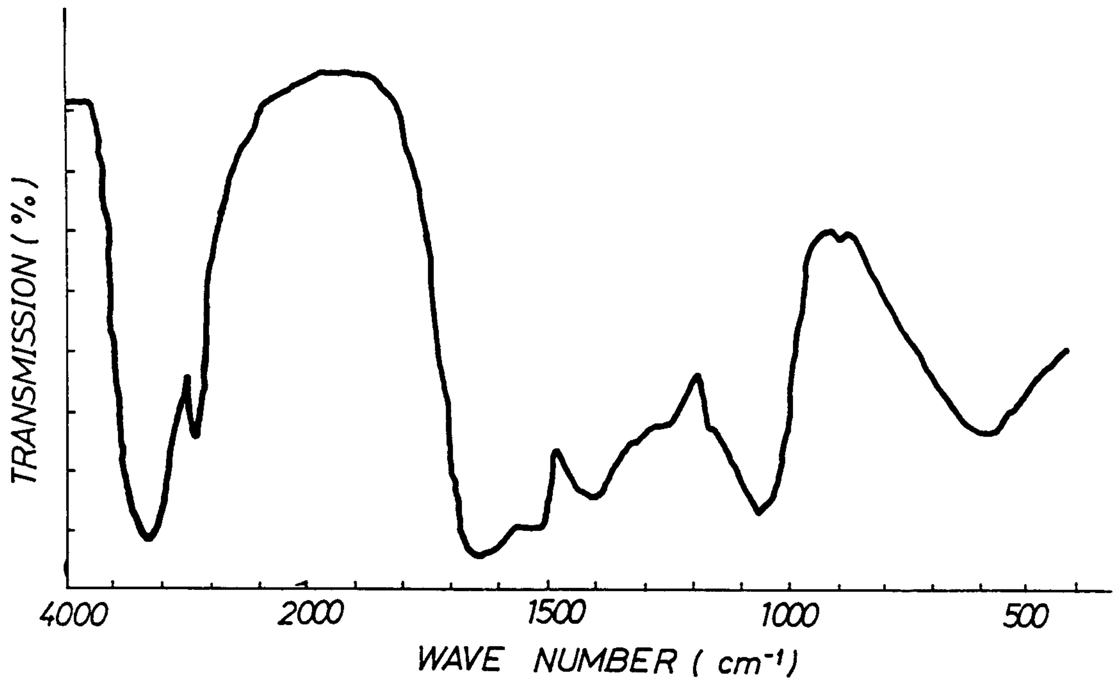
TRANSMISSION ( % )
4000
2000
1500
1000
500
WAVE NUMBER ( cm⁻¹)

Fig. 2

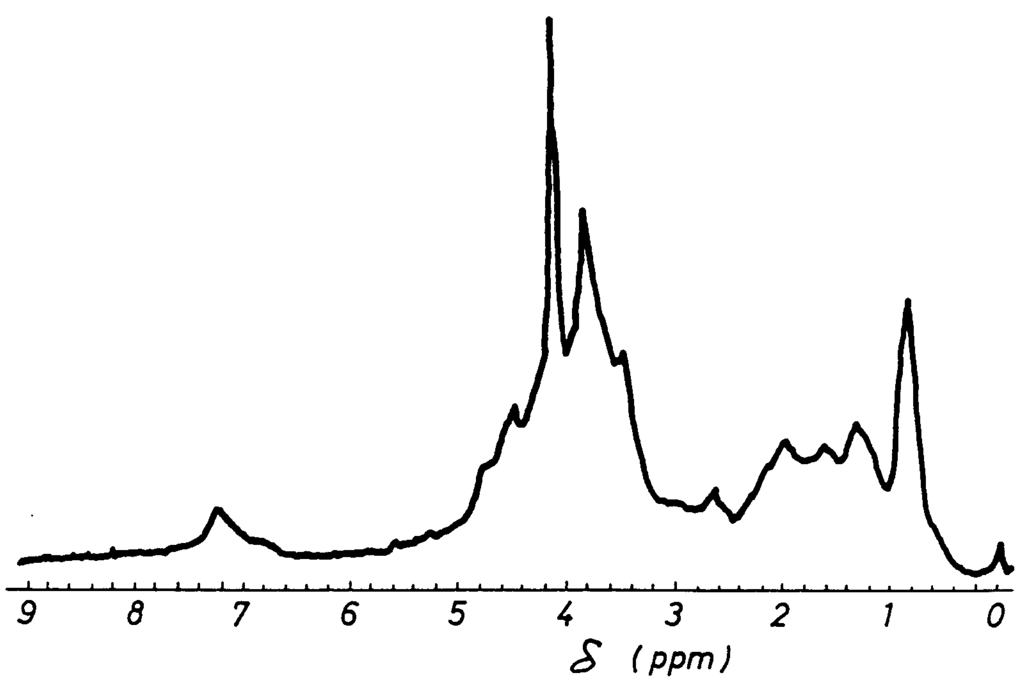
9
8
7
6
5
4
3
2
1
0
δ (ppm)